# EUROPEAN PATENT SPECIFICATION

(11) **EP 3 154 512 B1**
(45) Date of publication and mention of the grant of the patent: **24.07.2019**
(21) Application number: 15715682.9
(22) Date of filing: 15.04.2015
(51) Int. Cl.: A61K 9/00, A61K 9/14, A61K 9/20

(54) **SOLID ORAL FORMULATIONS COMPRISING SOLID MELT DISPERSIONS OF ORGANIC ACIDS IN XYLITOL**
FESTE ORALE FORMULIERUNGEN MIT FESTEN SCHMELZDISPERSIONEN ORGANISCHER SÄUREN IN XYLIT
FORMULATIONS ORALES SOLIDES COMPRENANT DES DISPERSIONS FONDUES SOLIDES D'ACIDES ORGANIQUES DANS DU XYLITOL

(30) Priority: 01.05.2014 EP 14166788
(43) Date of publication of application: 19.04.2017
(73) Proprietor: Hermes Arzneimittel GmbH, 82049 Pullach (DE)
(72) Inventor: BARTHOLOMÄUS, Johannes, 52080 Aachen (DE); DANDL, Klaus, 82041 Oberhaching (DE); SCHWEITZER, Thomas, 66589 Wemmetsweiler (DE); HÖBART, Hans, 82049 Pullach (DE)
(74) Representative: Pharma Patents International AG
(86) International application number: PCT/EP2015/000791
(87) International publication number: WO 2015/165571

(56) References cited:
- EP-A1- 0 415 326
- EP-A1- 2 441 436
- WO-A1-02/098387
- WO-A2-2005/063203
- US-A- 6 086 854
- US-A1- 2002 068 038
- US-B1- 6 197 787

## Description

### FIELD OF THE INVENTION

The present invention relates to the field of pharmaceutics and concerns drug formulations, their manufacture and their use.

### BACKGROUND OF THE INVENTION

To ensure patient compliance and acceptance, orally administered solid pharmaceutical compositions generally must be easily swallowed and should have no bitter taste or other undesirable organoleptic properties such as unpleasant mouth feel. This is particularly important for pharmaceutical compositions formulated into solid oral dosage forms such as oral granules, chewable or orodispersible tablets, which are aimed to be taken by the patient without the use of water or other liquids as a swallowing aid. These rely rather on the action of the patient's saliva to sufficiently dissolve, disintegrate or at least wet the dry components of the composition for swallowing.

Salivation-inducing agents may be incorporated to facilitate such processes, and are important especially in cases where the solid pharmaceutical composition is targeted for use in the treatment of patient groups with impaired or reduced
salivation, or where out of necessity the dosage form quantities are large.

For example, the patent application WO 2012/048846 describes pharmaceutical compositions in the form of pourable granules or chewable tablets comprising calcium salts (e.g. calcium acetate) and magnesium salts (e.g. magnesium carbonate) as phosphate-binding active ingredients which can be taken by the patients with renal insufficiency without the need for additional fluid or water. These incorporate, for the purposes of salivation induction and taste- masking, an effervescent agent, i.e. an alkaline or alkaline earth carbonate or bicarbonate in combination with an organic acid or organic acid salt. The foaming and carbon dioxide that is generated from the acid-base reaction that takes place in the mouth between the effervescent components is described to stimulate salivation, and to provide a pleasant and fresh feeling in the mouth. The effervescent agent is prepared by conventional methods in the art, such as by granulating the carbonate and the acid and drying the granules.

In view of long-term storage, a disadvantage of incorporating the effervescent as such is that the organic acid component will react with basic active ingredients or with other formulation components of a basic nature over time, and particularly in the presence of moisture. It is mentioned in WO 2012/048846 that good drying is essential as part of the process for preparing granules of the effervescent agent. A premature loss of effect and changes in the physical properties of both the organic acid, which inherently may also be effective for inducing salivation, and its reacting partner (in this case possibly also the API) is undesirable.

Maintaining a separate environment between the organic acid and a basic or acid-sensitive active ingredient is therefore desirable when the co-formulation of such components is contemplated. The separation of incompatible components by physical barriers such as tablet or coating layers is generally known in the art. Further considerations, such as with respect to the dissolution of the component that is coated are however required; these may negatively interfere i.e. retard release.

The patent EP0415326B1 discloses 'foaming' (i.e. effervescent) compositions comprising a carbonate salt, such as calcium carbonate, and an organic acid of which the crystal surface is coated with a sugar. The coating of the organic acid is performed so as to prevent the premature effervescence, i.e. reaction of the acid with the carbonate component, and on the premise that due to its water solubility, the sugar coating would not significantly delay the dissolution of the organic acid. EP0415326B1 teaches the coating of the crystal surface of the organic acid through a process of spray-wetting of the crystal surfaces with water followed by charging the apparatus/spraying with a sugar (e.g. sucrose or lactose), using equipment such as a centrifugal coating apparatus with a fluidized bed.

A disadvantage of such a process of coating however is that the distribution of the sugar on the acid surface may not be uniform, such that the whole available acid crystal surface become fully covered by a sugar film or particle. Partial or
incomplete coating may render the acid crystal core of such particles vulnerable to exposure to the basic components in the composition, and may facilitate erosion of the sugar coating by moisture. Additional processing steps are also required, such as drying to remove residual moisture since the wetting of the organic acid particles with water is involved. It is mentioned in the patent that the procedure of wetting by spraying water, and spraying of sugar may be necessarily repeated (thus increasing the required processing times) until a desired amount of sugar is coated onto the organic acid crystals.

Specific examples in EP0415326B1 only mention citric acid and tartaric acid as the organic acids; the sugars lactose and sucrose are used for the coating. The formulation of such organic acids is also described only in the context of compositions containing calcium salts. No teaching is provided with respect to co- formulation with therapeutically active combinations of a magnesium salt with a calcium salt.

It is therefore an object of the invention to provide improved methods for overcoming the limitations and disadvantages of the prior art methods for the co- formulation of organic acid with basic active ingredients, and which allows for rapid dissolution of the organic acid within a patient's mouth directly after oral administration. A further object of the invention is to provide improved formulations of organic acids which are compatible with basic compounds, and methods for preparing such formulations. Another object of the invention is to provide a selection of suitable organic acids that do not compromise the binding of phosphate by calcium and/or magnesium salts as well as a method of in-vitro phosphate binding testing for selection of suitable organic acids.

These and other objects which will become clear on the basis of the description and claims are achieved by the subject-matter as defined in the independent claims below, with particular embodiments outlined in the dependent claims.

### SUMMARY OF THE INVENTION

In a first aspect, the invention provides a method for preparing a solid composition comprising an organic acid, the method comprising a step of dispersing the organic acid in molten xylitol such as to form a mixture, and subsequently allowing the mixture to solidify, wherein the organic acid is provided in powder form. The organic acid is preferably selected from citric acid, fumaric acid, tartaric acid, malic acid, succinic acid and ascorbic acid. In a particular embodiment, the organic acid is fumaric acid.

In a further aspect, the invention provides a solid composition which is obtainable by the method comprising a step of dispersing an organic acid in molten xylitol such as to form a mixture, and subsequently allowing the mixture to solidify, wherein the organic acid is provided in powder form.

In a further aspect, the invention provides a solid dispersion of an organic acid in xylitol, wherein the organic acid is selected from citric acid, fumaric acid, tartaric acid, malic acid, succinic acid and ascorbic acid. In one of the preferred embodiments, the organic acid is fumaric acid. The solid dispersion is optionally in the form of multiple units.

In further aspect, the invention provides a solid pharmaceutical composition comprising at least one therapeutically active ingredient selected from a calcium or a magnesium salt or a combination thereof, and an organic acid provided in form of a solid dispersion in xylitol selected from ascorbic acid, fumaric acid, malic acid and any combinations thereof.

In yet a further aspect, the invention provides a solid pharmaceutical composition comprising at least one therapeutically active ingredient selected from a calcium or a magnesium salt or a combination thereof, and a solid composition of an organic acid in molten xylitol obtainable by the method of the first aspect of the invention or a solid dispersion of an organic acid in xylitol, wherein the organic acid is selected from citric acid, fumaric acid, tartaric acid, malic acid, succinic acid and ascorbic acid. Further aspects and embodiments of the invention will become clear on the basis of the detailed description, including the examples, and the patent claims.

### DETAILED DESCRIPTION OF THE INVENTION

In a first aspect, the invention provides a method for preparing a solid composition comprising an organic acid. The method is characterised in that it comprises a step of dispersing the organic acid in molten xylitol such as to form a mixture, and subsequently allowing the mixture to solidify, wherein the organic acid is provided in powder form. The organic acid is preferably selected from citric acid, fumaric acid, tartaric acid, malic acid, succinic acid and ascorbic acid. In a particular embodiment, the organic acid is fumaric acid.

In a further aspect, the invention provides a solid composition which is obtainable by the method comprising a step of dispersing an organic acid in molten xylitol such as to form a mixture, and subsequently allowing the mixture to solidify, wherein the organic acid is provided in powder form.

The inventors have surprisingly found that organic acids may be easily and cost-effectively formulated according to this method. The processing times are short, and the required energy is relatively low as no drying step is needed. Unexpectedly, the method does not lead to any significant degree of esterification of the organic acid. Moreover, it was found that the product obtained from the method exhibits a surprisingly rapid release of the organic acid upon contact with water or an aqueous medium while at the same time being substantially inert to basic compounds that are mixed or co-formulated with the product. Thus, the invention provides several important advantages over the prior art methods of formulating organic acids.

It is believed that the method of the invention typically leads to the formation of a solid dispersion of the organic acid in xylitol, as further described below. However, it may also be possible that simply an intimate mixture of the two components is created.

The xylitol appears to provide a physical barrier between the organic acid and the external environment, thus preventing the acid from reacting with e.g. any basic active ingredients and/or excipients that may be mixed or co-formulated with the product of the method of the invention. The use of xylitol, as opposed to alternative carriers, allows for fast dissolution and ensures that the resulting particles readily dissolve and release the organic acid, when orally administered, directly within the mouth of the patient. Xylitol, moreover, can simultaneously function as a sweetener and contribute to better taste; with a negative heat of solution it may also impart a cooling and refreshing feeling in the mouth.

An organic acid, in context of the invention, refers to an organic compound with acidic properties, preferably such as an organic compound with one or more carboxyl functional groups. Particularly preferred are the commonly termed 'food' or 'edible' acids, that is, organic acids such as citric acid, fumaric acid, tartaric acid, malic acid, succinic acid, ascorbic acid, lactic acid, adipic acid, etc., or combinations
thereof. In a preferred embodiment, the organic acid is selected from citric acid, fumaric acid, tartaric acid, malic acid, succinic acid, and ascorbic acid. As used herein, organic acid includes also pharmaceutically acceptable salts, hydrates, and crystal polymorphs. In one particular embodiment, the organic acid is fumaric acid.

The amount of organic acid relative to that of the xylitol is typically selected to yield a content of the organic acid in the mixture with xylitol in the range from about 1 wt.-% to about 60 wt.-%. More preferably, the content of the organic acid in the mixture is from about 2 wt.-% to about 50 wt.-%, such as from about 5 wt.-% to about 40 wt.-%. These ranges also apply in the case of fumaric acid being selected as the organic acid.

Moreover, the method is typically conducted by performing the steps of (a) melting xylitol, (b) dispersing the organic acid in the molten xylitol such as to form a mixture, and subsequently (c) allowing the mixture to solidify. The organic acid is provided in powder form. Preferably, at least the step of dispersing the organic acid in the molten xylitol is conducted under stirring.

The method may be conducted using any suitable equipment, such as a melting vessel equipped with a mixer. The mixer is preferably capable of exerting high shear. The xylitol is charged to the vessel and heated, typically under mixing, until melted. The organic acid, if not already charged to the vessel before or along with the xylitol, is added and dispersed in the xylitol melt, preferably while mixing. Subsequently, the mixture is poured e.g. into a tray where it is allowed to cool and solidify.

Alternatively, the method may also be conducted using melt extrusion equipment. According to this embodiment, a mixture of the organic acid and the xylitol is heated under pressure, whereby the xylitol melts and the acid is dispersed in the molten xylitol. The mixture is extruded and allowed to solidify.

Further processing, as necessary, of the resulting solid dispersion product is also within the scope of the invention, for example grinding or milling of the product using conventional equipment such as a knife mill, ball mill, hammer mill, roller mill, colloidal mill, jet mill or the like, or sieving to select certain unit or
particle sizes. Particle size reduction may be performed in two steps, such as crushing into coarse particles followed by milling and optionally sieving, e.g. using a sieve with a mesh width of about 300 to 800 µm.

In a preferred embodiment of the invention, the melt-processing temperature is less than 180 °C, and more preferably less than 160 °C. In further preferred embodiments, the xylitol is heated to not more than about 150 °C, or 140 °C, or 130 °C, or 120 °C, respectively. Particularly preferred are melt-processing temperatures of at least 91 °C but no higher than 120 °C., such as from about 92 °C to about 115 °C, or from about 92 °C to about 110 °C, respectively. As used herein, the melt processing temperature is the temperature of the melted xylitol in the presence of the organic acid.

Optionally, one or more further compounds, such as pharmaceutically acceptable excipients, may be added to the mixture, either before the addition of the organic acid or thereafter.

It may be useful to provide the xylitol which is to be melted in the form of fine particles, such as with a mass mean diameter of less than about 800 µm, in particular not more than about 500 µm, or not more than about 350 µm, or not more than 250 µm, respectively, for economic reasons and/or in order to facilitate the formation of a melt.

In one of the preferred embodiments, the method is conducted by performing the steps of (a) melting xylitol, (b) dispersing the organic acid in the molten xylitol such as to form a mixture, followed by (c) cooling the mixture to a temperature below about 94°C, followed by (d) adding crystalline xylitol to the mixture before the mixture has solidified, followed by (e) allowing the mixture to solidify.

It has been surprisingly found that the processing times can be substantially reduced by the addition of a relatively small amount of crystalline xylitol in step (d), i.e. after the mixture has been cooled below the melting point of xylitol, but before it has solidified. The degree of process acceleration is remarkable; the reduction in processing time may be more than 80%, or even more than 90%,
depending on the circumstances. Without being bound to theory, it is believed that the additional xylitol catalyses or triggers the solidification or crystallization process of the solid dispersion.

In one particular embodiment, the xylitol added during cooling, or after the initial cooling of the mixture, has a particles size distribution with a mass mean diameter of at least about 100 µm, or at least about 150 µm, or even greater. The additional xylitol particles may be added after the mixture is cooled to about 88-92 °C and/or while the mixture is still liquid.

In another aspect, the invention provides a solid dispersion of an organic acid in xylitol. Such solid dispersion is useful for the preparation of solid, oral pharmaceutical compositions, in particular for the formulation of direct-to-mouth granules. Again, in one of the preferred embodiments, the organic acid is selected from citric acid, fumaric acid, tartaric acid, malic acid, succinic acid, and ascorbic acid. In a further preferred embodiment, the acid is fumaric acid.

As understood herein, a solid dispersion refers to a solid material wherein a first component is molecularly or colloidally dissolved, or finely dispersed, in a second component which forms a continuous phase. In the case of the invention, a solid dispersion of an organic acid in xylitol means that the organic acid is dispersed or dissolved in a matrix or carrier, i.e. a continuous phase, comprising xylitol. It is not excluded that other components such as further excipients, other than the organic acid may be present and dispersed in the xylitol. The matrix can be either crystalline or amorphous. The dispersed component, for example the organic acid, may be dispersed molecularly, as amorphous particles, or in crystalline form. The term solid dispersion as used herein should be understood as covering all forms of solid dispersions known in the art i.e. any chemical and physical arrangements of the dispersed component with respect to the solid state matrix or carrier or continuous phase, for instance solid solutions, glass solutions and suspensions, crystalline or amorphous solid dispersions.

The solid dispersion of the invention may be in the form of multiple units. As understood herein, the term 'multiple units' refer to a plurality of individual crystals or particles, or particles agglomerated in the form of large units such a granules, microgranules, pellets, micropellets, spherules, beads or microtablets. In a specific embodiment, the solid dispersion of the invention is in the form of particles or microgranules. Preferably, the particles or microgranules have a particle size distribution with a mass mean diameter of less than 1,500 µm, and more preferably of less than about 1,000 µm. Optionally, the mass mean diameter may also be less than about 500 µm, with the majority of the particles or microgranules having a diameter of about 100 µm to about 600 µm.

As mentioned, the solid dispersion of the invention may be prepared by the method described above, i.e. comprising a step of dispersing the organic acid in molten xylitol, such as to form a mixture, and subsequently allowing the mixture to solidify, wherein the organic acid is provided in powder form.

While melt-processing is preferred, the solid dispersion may also be prepared by other methods, such as solvent-based processes in which the xylitol is dissolved in an appropriate solvent, and the organic acid is dispersed in the xylitol solution, followed by the evaporation of the solvent. Such process may, for example, be conducted in a spray dryer.

Alternatively, the solid dispersion may be prepared by a method based on near-critical or supercritical fluid technology.

According to a further aspect of the invention, a solid pharmaceutical composition is provided which comprises a) at least one therapeutically active ingredient selected from a calcium or magnesium salt or a combination thereof, and b) an organic acid provided in form of a solid dispersion in xylitol, wherein the organic acid is selected from ascorbic acid, fumaric acid, and malic acid. Optionally, two or more of these organic acids are present in the composition.

As used herein, a pharmaceutical composition is a composition comprising at least one therapeutically active ingredient in combination with at least one pharmaceutical excipient. The pharmaceutical excipient is, in the amount that it is incorporated into the composition, pharmacologically inert. A therapeutically active ingredient refers to any compound or mixture of compounds that is capable of and useful for producing a desired pharmacological effect, i.e. having a biological activity useful for the therapy, management, prevention or diagnosis of a disease, health condition or symptoms. Synonyms for active ingredient include, without
limitation, bioactive compounds, drug substance, active pharmaceutical ingredient (API), active or pharmacological agent, etc.

Calcium and magnesium salts, which may be used as active ingredients may react with acidic excipients such as organic acids. These salts are useful, when taken orally, for binding phosphate and preventing its gastrointestinal uptake and as such for controlling serum phosphate levels. Organic acids that are present in a solid pharmaceutical composition together with these salts as such may compete with the phosphate binding, and/or render these salts unavailable for phosphate binding. At the same time, however, in many cases the inclusion of an organic acid into oral preparations would be highly useful, for instance as a salivation promoting agent.

It has been found by the inventors that organic acids selected from ascorbic acid, fumaric acid or malic acid, may be compatibly combined with a solid composition comprising calcium and magnesium salts. As further detailed below, it has been found that malic acid, ascorbic acid and fumaric acid, in particular, do not significantly compete and interfere with the binding of phosphate of calcium and magnesium salts, or only to a substantially reduced degree. Unlike other organic acids, these also do not rapidly form complexes or water-insoluble precipitates nor do they form poortasting complexes with the calcium and magnesium active ingredients.

In one of the preferred embodiments of the invention, a the solid pharmaceutical composition is provided which comprises at least one therapeutically active ingredient selected from a calcium or a magnesium salt or a combination thereof and an organic acid in molten xylitol, wherein the organic acid is selected from ascorbic acid, fumaric acid, or and malic acid, or a combination thereof. The calcium salt may be calcium acetate, and the combination of a calcium and a magnesium salt may be a combination of calcium acetate and magnesium carbonate. As mentioned, it has been found by the inventors that organic acids selected from ascorbic acid, fumaric acid or malic acid, in contrast to citric acid and other acids commonly used as acid components in oral drug formulations, do not significantly compete and interfere with the binding of phosphate of calcium and magnesium salts.

According to a further aspect of the invention, a solid pharmaceutical composition is provided which comprises a) at least one therapeutically active ingredient selected from a calcium or magnesium salt or a combination thereof, and b) a solid dispersion of an organic acid in xylitol, wherein the organic acid is selected from citric acid, fumaric acid, tartaric acid, malic acid, succinic acid and ascorbic acid, or a solid composition obtainable from a method comprising the step of dispersing an organic acid in molten xylitol such as to form a mixture, and subsequently allowing the mixture to solidify, wherein the organic acid is provided in powder form. In one of the preferred embodiments, the solid pharmaceutical composition comprises a) a therapeutically active combination of a calcium and magnesium salt and b) a solid dispersion of fumaric acid in xylitol.

The inventors have found that solid pharmaceutical compositions comprising therapeutically active ingredients of a basic nature such as calcium or a magnesium salts, or a combination thereof may also be advantageously formulated with an organic acid, when the organic acid is incorporated into the composition as a solid dispersion in xylitol, i.e. in the form of a solid dispersion with xylitol as the continuous phase. This permits for a wider range of organic acids to be incorporated into such compositions. Such compositions, moreover, are even more stable over time, i.e. the organic acid is does not readily contact and react with the basic active ingredients, such as during storage and prior to administration to a patient, to form salt complexes and other unintended by- products.

Accordingly, the invention provides a method of preparing a solid pharmaceutical composition comprising one or more basic therapeutically active ingredients and an organic acid, comprising the step of formulating said active ingredient with a solid dispersion of the organic acid in xylitol. In the context of the current invention, a basic therapeutically active ingredient is to be understood as a pharmaceutically active compound that has basic properties and that may be sensitive to acid and/or undergo acid-base reaction, when in direct contact with an organic acid component of the composition.

In a particularly preferred embodiment of the invention, the basic therapeutically active ingredient is selected from a calcium or magnesium salt or a combination thereof. As used herein, the term salt includes any hydrates, amorphous forms and crystalline polymorphs forms. Preferably the pharmaceutical composition of the invention comprises at least one therapeutically active ingredient selected from a calcium salt, wherein the calcium salt is selected from calcium acetate, calcium carbonate, or calcium chloride. In another preferred embodiment, the calcium salt is calcium acetate. Pharmaceutical compositions comprising magnesium carbonate as active ingredient are also preferred.

In a further preferred embodiment, the solid pharmaceutical compositions comprise a combination of calcium salt and magnesium salt, preferably calcium acetate and magnesium carbonate. Optionally, the weight ratio of calcium salt to magnesium salt is from about 1:1 to 4:1. Other preferred weight ratios of the calcium salt to magnesium salt are in the range from about 1.5:1 to about 2:1. The molar ratio between the calcium and the magnesium may optionally be in the range from about 5:1 to about 1:5, or from about 4:1 to about 1:4, or from about 3:1 to about 1:3, of from about 1.5:1 to about 1:1.5, respectively.

The therapeutically active ingredients may be incorporated into the solid pharmaceutical composition as such, in the form of crystals or particles of any size, but also in the form of larger structural units, such as granules, microgranules, pellets and other types of agglomerated particles, which may be formed by methods known in the art such as wet granulation, dry granulation or melt-granulation of the active ingredient together with additional excipients.

According to another embodiment of the invention, the solid pharmaceutical composition comprises at least one calcium salt, wherein the calcium salt or granule comprising calcium salt is coated with a taste-masking compound. Taste-masking compounds generally known in the art are within the scope of the invention; these include, but are not limited to, lipophilic excipients such as lipids, waxes, fatty acids, fatty alcohols, fatty acid esters, triglycerides, partial glycerides, fatty alcohol polyglycerols, fatty acid PEG esters, fatty acid alcohol-PEG-ethers or mixtures thereof; hydrophobic or hydrophilic polymeric excipients such as Eudragits, hydroxypropyl methyl cellulose and other hydroxylalkyl celluloses, polyacrylic acids, polyacrylates, polymethacrylates, polyvinylpyrrolidone, cellulose acetate, cellulose acetate butyrate, or mixtures thereof.

The taste-masking coating comprising the taste-masking compound can be applied using coating methods useful and known in the art such as spray coating or hot-melt coating, and may be applied directly to the therapeutically active ingredient particle or to a granule or any other unit comprising the therapeutically active ingredient and may comprise other excipients as necessary. The coating is understood as one or more layers of material substantially enclosing the active ingredient, or at least the majority of the active ingredient (or a unit comprising the active ingredient).

The solid pharmaceutical composition of the invention may be formulated into various types of solid oral dosage forms. Particularly useful embodiments are solid pharmaceutical formulations which comprise of multiple units, or which disintegrate in the mouth of the patient into multiple units such as direct-to-mouth granules, a powder for oral use, or orally disintegrating tablets.

As used herein, direct-to-mouth granules (also referred to as oral granules, instant granules or pourable granules) are designed for direct oral administration without water, or with minimal water. Direct-to-mouth granules may represent mixtures of various types of multiple units, which units may be agglomerated and/or non-agglomerated particles. Powders for oral use are also within the scope of the invention. Compared to granules, powders are generally understood to comprise multiple units with smaller or finer particle sizes. Preferably, the powder is free-flowing and may also be administered without the aid of water or another fluid, e.g. from a stick-pack or sachet directly into a patient's mouth by pouring. Such direct-to-mouth compositions may represent mixtures of the solid pharmaceutical composition of the invention with other excipients such as additional sweeteners or flavourings, any of which may be agglomerated or granulated.

In a preferred embodiment, a solid pharmaceutical composition of the invention comprising a) calcium acetate or magnesium carbonate, or a combination of calcium acetate and magnesium carbonate, as therapeutically active ingredients and b) a solid dispersion of fumaric acid in xylitol is formulated as direct-to-mouth granules or as a powder for oral use.

An orally disintegrating tablet may be defined as solid single-unit dosage forms that rapidly disintegrate, such as into multiple units, in the mouth of a patient without the need for chewing, typically within less than about one or two minutes. Orally disintegrating tablets are usually pressed with lower compression forces than conventional tablets to obtain higher porosity. For tablets which contain a higher amount of moisture or a sublimable excipient, alternatively their porosity may be increased by drying or sublimation step. Orally disintegrating tablets may further include optimized amounts of disintegrants, such as crosslinked polymers, low-substituted celluloses, or highly water-soluble excipients and salivation-inducing agents to further contribute to rapid disintegration.

Optionally, the solid pharmaceutical composition of the invention may comprise one or more further excipients commonly used in oral drug formulations, such as one or more glidants, flow regulators, bulking agents, colouring agents, antioxidants, pH-modifiers, sweeteners, flavouring agents, lubricants, and the like.

In another embodiment of the invention, the solid pharmaceutical compositions of the invention may be used for the treatment of patients with renal insufficiency. Renally insufficient patients are those suffering renal conditions and/or renal failure, and inlude patients requiring dialysis. As understood herein, the term treatment refers to the therapy, the management or the prevention of a condition, for example, in the case of renally insufficient patients, the condition of hyperphosphatemia, or elevated blood phosphate levels. The progression of chronic kidney disease is defined by the loss in glomerular filtration rate and subdivided in stages from CKD stage 1 (normal kidney function) to CKD stage 5 (end stage renal disease). A definition of the CKD stages can be found in literature. (K/DOQI clinical practice guidelines for chronic kidney disease: evaluation, classification, and stratification. Kidney Disease Outcome Quality Initiative. Am J. Kidney Dis 2002;39:S1-S246) The solid pharmaceutical compositions of the invention may, e.g., be used for the treatment or the prevention of
hyperphosphatemia associated with chronic renal insufficiency in patients undergoing hemodialysis or peritoneal dialysis (CKD stage 5, end stage renal disease, ESRD). The solid pharmaceutical compositions of the invention may also be used for the treatment or the prevention of hyperphosphatemia associated with chronic renal insufficiency in patients in CKD stage 4 or earlier.

In a preferred embodiment of the invention, a solid pharmaceutical composition comprising a) one or more phosphate-binding compounds, such as selected from calcium or magnesium salts, or a combination thereof, and b) an organic acid provided in form of a solid dispersion in xylitol, wherein the organic acid is selected from ascorbic acid, fumaric acid and malic acid is used for the treatment of renally insufficient patients. The solid pharmaceutical compositions of the invention may, e.g., be used for the treatment or the prevention of hyperphosphatemia associated with chronic renal insufficiency in patients undergoing hemodialysis or peritoneal dialysis (CKD stage 5, end stage renal disease, ESRD). The solid pharmaceutical compositions of the invention may also be used for the treatment or the prevention of hyperphosphatemia associated with chronic renal insufficiency in patients in CKD stage 4 or earlier. Such solid pharmaceutical compositions may comprise only calcium salt as the phosphate-binding compound, for example calcium acetate. A preferred calcium and magnesium salt combination is calcium acetate and magnesium carbonate.

In another preferred embodiment of the invention, a solid pharmaceutical composition comprising a) one or more phosphate-binding compounds, such as a combination of a calcium and a magnesium salts, and b) a solid dispersion of an organic acid in xylitol is used for the treatment of renally insufficient patients. Further embodiments, options and/or preferences are illustrated by the following examples.

### EXAMPLES

### Example 1 - Solid dispersion of fumaric acid in xylitol

| **Component** | **Total weight (g)** |
|---|---|
| Xylitol (crystalline, fine) | 189.00 |
| Fumaric acid | 21.00 |

A mixer was charged with fine crystalline xylitol and heated. Fumaric acid was added to the melted xylitol at approx. 104 °C while stirring at 1100 rpm. The resulting dispersion was transferred to a tray and cooled to RT (23°C). The resulting solid dispersion was milled and then sieved (0.8 mm).

The solidification (or crystallization) of the solid dispersion was completed in ca. 2 days. No fumaric acid ester by-products were evident.

### Example 2- Solid dispersion of fumaric acid in xylitol

| **Component** | **Total weight (g)** |
|---|---|
| Xylitol (crystalline, approx. 90 µm) | 185.00 |
| Fumaric acid | 21.00 |
| Xylitol (crystalline, approx. 200 µm) | 4.00 |

A mixer was charged with fine crystalline xylitol and heated. Fumaric acid was added to the melted xylitol at approximately 98 °C while stirring at 1100 rpm. The dispersion was cooled to approximately 92 °C, followed by addition of the coarse crystalline xylitol. The dispersion was transferred to a tray and then cooled to RT. Solidification of the solid dispersion was completed in ca. 1 hour. The resulting solid dispersion was milled and then sieved (0.8 mm). Sample content analysis indicated a content of 99.5% (HPLC) and 101% (acidimetry) of fumaric acid, respectively.

The addition of the crystalline xylitol to the dispersion melt at a temperature about 2-3 °C below the melting point of xylitol drastically reduces the process time required for the dispersion to solidify to a state suitable for milling.

### Comparative Example 1 - Solid dispersion of fumaric acid in sorbitol/mannitol

| **Component** | **Total weight (g)** |
|---|---|
| Sorbitol Mannitol | 6.00 |
| Fumaric Acid | 183.00 |
| | 21.00 |

A mixer was charged with the mannitol and sorbitol and heated. Fumaric acid was added to the mannitol-sorbitol melt at 170-180°C while stirring at 1200 rpm. The dispersion was transferred to a tray and then cooled to RT. The resulting solid dispersion was milled and then sieved (0.8 mm). Sample content analysis indicated the presence of up to 50 wt. % fumaric esters.

Higher processing temperatures significantly increase the amount of fumaric ester by-products, especially when mixing time at those temperatures is prolonged. The high content of fumaric acid esters in the resulting solid dispersion also increases its plasticity so that milling is difficult or not feasible.

### Example 3 - Production-scale preparation of a solid dispersion of fumaric acid in xylitol

| **Component** | **Total weight (kg)** |
|---|---|
| Xylitol (crystalline, fine) Fumaric | 112.5 |
| acid | 13.0 |
| Xylitol (crystalline, coarse) | 4.5 |

A melting kettle equipped with a stirring unit was charged with fine crystalline xylitol and heated. Fumaric acid was added to the melted xylitol at 92°C while stirring. The dispersion was cooled to 90 °C, followed by addition of the coarse crystalline xylitol. The dispersion was transferred onto a cooling conveyor and allowed to cool to RT. Crystallization of the solid dispersion was completed in ca. 1.5 hours. The resulting solid dispersion was then milled and sieved (1.5 mm).

### Example 4 - Preparation of direct-to-mouth granules

| **Component** | **Preferred range [wt.-%]** |
|---|---|
| Calcium acetate (coated) | 10-50 (e.g. 40) |
| Magnesium carbonate | 10-50 (e.g. 15) |
| Fumaric acid/xylitol solid dispersion (of Example 3) | 1-50 (e.g. 10) |
| Mannitol | 0-50 (e.g. 10) |
| Xylitol | 0-20 (e.g. 5) |
| Sorbitol | 0-50 (e.g. 10) |
| Maltodextrin | 0-30 (e.g. 5) |
| Flavour | 0-10 (e.g. 3) |
| Carmellose sodium | 0-10 (e.g. 2) |

A direct-to-mouth granule composition was prepared by combining the solid dispersion of fumaric acid in xylitol with the active ingredients calcium acetate (lipid-coated for taste masking according to WO 2010/070028; alternatively, e.g. calcium acetate coated with a subcoat of hypromellose and an overcoat of Eudragit® E or Eudragit® RL/RS may be used) and magnesium carbonate and the other excipients as listed above.

Studies to test the stability of this granule composition were conducted. Samples of were stored at 40 °C for two weeks and at 55 °C for 2 days. In both experiments, no generation of carbon dioxide gas was observable (from the reaction of fumaric acid with the magnesium carbonate active ingredient).

### Example 5 - Phosphate-binding test

An *in-vitro* study was conducted to evaluate the phosphate binding activity of the active ingredients, calcium acetate and magnesium carbonate in the commercially available tablet OsvaRen® in the presence of organic acid excipients. OsvaRen® is a tablet with a film-coating of castor oil and hypromellose and a core containing calcium acetate, magnesium carbonate, and as excipients, starch, pregelatinized maize starch, sucrose, gelatin, croscarmellose sodium and magnesium stearate.
*Sample 1* - *OsvaRen*® *tablet (control).* A solution of 12.5 mmol of ammonium dihydrogen phosphate in 250 ml of imidazole buffer, pH 7, was prepared. One tablet of OsvaRen® was added. After 2 hours of equilibration time, the bound phosphate concentration of the solution was determined as the difference between 12.5 mmol of phosphate and the concentration of free phosphate in solution as detected by ion exchange chromatography.
*Sample 2* - *OsvaRen*® *tablet and citric acid and monosodium citrate.* The experiment was conducted according the procedure described for the control sample, but with one OsvaRen® tablet and 50 mg of citric acid and 100 mg of monosodium citrate (i.e. total 0.727 mmol of acid).
*Sample 3* - *OsvaRen*® *tablet and malic acid.* The experiment was conducted according the procedure described for the control sample, but with one OsvaRen® tablet and 97.5 mg (0.727 mmol) of malic acid.
*Sample 4* - *OsvaRen*® *tablet and fumaric acid.* The experiment was conducted according the procedure described for the control sample, but with one OsvaRen® tablet and 84.4 mg (0.727 mmol) of fumaric acid.
*Sample 5* - *OsvaRen*® *tablet and mixture of fumaric acid and malic acid* (1:1). The experiment was conducted according the procedure described for the control sample, but with one OsvaRen® tablet and 42.2 mg (0.3635 mmol) of fumaric acid and 48.7 mg (0.3635 mmol) of malic acid.
*Sample 6* - *OsvaRen*® *tablet and ascorbic acid.* The experiment was conducted according the procedure described for the control sample, but with one OsvaRen® tablet and 128 mg (0.727 mmol) of ascorbic acid.
*Sample* 7 - *OsvaRen*® *tablet and citric acid, monosodium citrate and granule excipients based on sugar alcohols, maltodextrin, flavour, and carmellose sodium.* An OsvaRen® tablet, citric acid, monosodium citrate, and 0.8223 g of an excipient mixture for direct-to-mouth granules was used.

The results show that citric acid and/or citrate reduce the level of phosphate binding by about 27% (Sample 2) or 25% (Sample 7). It is presumed,
without wishing to be bound by theory, that the citric acid/citrate forms relatively stable calcium and/or magnesium chelates and decreases the availability of the calcium and/or cation for binding with phosphate. As indicated by the similar results for Sample 2 and Sample 7, the presence of further excipients commonly used in granule formulations has little or no effect on this. In contrast, phosphate binding was reduced only by 3% in the presence of ascorbic acid, and only by 5% in the presence of fumaric acid relative to the control Osvaren® tablet, indicating that ascorbic acid, malic acid and fumaric acid are much more useful as acid components in formulations with calcium and/or magnesium salts as active ingredients for phosphate binding.

| **Sample** | | **Bound Phosphate** |
|---|---|---|
| Sample 1: | Osvaren® tablet | 5.07 ± 0.06 |
| Sample 2: | Osvaren® tablet + citric ac-citrate | 3.70 ± 0.02 |
| Sample 3: | Osvaren® tablet + malic acid | 4.25 ± 0.10 |
| Sample 4: | Osvaren® tablet + fumaric acid | 4.83 ± 0.06 |
| Sample 5: | Osvaren® tablet + fumaric acid/malic | 4.54 ± 0.06 |
| Sample 6: | Osvaren® tablet + ascorbic acid | 4.92 ± 0.06 |
| Sample 7: | Osvaren® tablet + citric ac-citrate + granule excipient mixture | 3.79 ± 0.29 |

## Claims

1. A method for preparing a solid composition comprising an organic acid, the method comprising a step of dispersing the organic acid in molten xylitol such as to form a mixture, and subsequently allowing the mixture to solidify,
wherein the organic acid is provided in powder form.

2. The method of claim 1, wherein the organic acid is selected from citric acid, fumaric acid, tartaric acid, malic acid, succinic acid and ascorbic acid.

3. The method of claim 2, wherein the organic acid is fumaric acid.

4. The method of claim 3, comprising the steps of
a) melting the xylitol,
b) dispersing the fumaric acid in the molten xylitol such as to form a mixture, followed by
c) cooling the mixture to a temperature below about 94°C, followed by
d) adding crystalline xylitol to the mixture before the mixture has solidified, followed by
e) allowing the mixture to solidify.

5. The method of claim 4, wherein the crystalline xylitol is added in step c) after the mixture is cooled to about 88-92 °C, and/or while the mixture is still liquid.

6. The method of any preceding claim, wherein the melt-processing temperature is at least 91 °C and less than 180 °C, preferably at least 91 °C but not higher than 120 °C.

7. A solid composition obtainable by the method of any one of claims 1 to 6.

8. A solid dispersion of an organic acid in xylitol, wherein the organic acid is selected from citric acid, fumaric acid, tartaric acid, malic acid, succinic acid and ascorbic acid.

9. The solid dispersion of claim 8, wherein the organic acid is fumaric acid.

10. The solid dispersion of claims 8 to 9, being in the form of multiple units.

11. A solid pharmaceutical composition comprising:
a) at least one therapeutically active ingredient selected from a calcium or a magnesium salt or a combination thereof, and
b) the solid composition of claim 7 or the solid dispersion of claims 8 to 10.

12. The solid pharmaceutical composition of claim 11, wherein the calcium salt is selected from calcium acetate, calcium carbonate, calcium chloride or a combination thereof.

13. The solid pharmaceutical composition of claim 12, wherein the calcium salt is calcium acetate and/or the magnesium salt is magnesium carbonate.

14. The solid pharmaceutical composition of claims 10 to 13, being in the form of granules, direct-to-mouth granules, a powder for oral use, a tablet, a chewable tablet, or an orally disintegrating tablet.

15. The solid pharmaceutical composition of claims 10 to 14 for use in the treatment or the prevention of hyperphosphatemia associated with chronic renal insufficiency in patients undergoing hemodialysis or peritoneal dialysis (CKD stage 5) or in patients with CKD stage 4 or earlier.

## Patentansprüche

1. Verfahren zur Herstellung einer festen Zusammen-setzung, die eine organische Säure umfasst, bei dem man die organische Säure in geschmolzenem Xylitol dispergiert, wobei man eine Mischung erhält, und die Mischung anschließend fest werden lässt, wobei die organische Säure in Pulverform bereit-gestellt wird.

2. Verfahren nach Anspruch 1, bei dem die organische Säure aus Citronensäure, Fumarsäure, Weinsäure, Äpfelsäure, Bernsteinsäure und Ascorbinsäure ausgewählt wird.

3. Verfahren nach Anspruch 2, bei dem es sich bei der organischen Säure um Fumarsäure handelt.

4. Verfahren nach Anspruch 3, bei dem man
a) das Xylitol aufschmilzt,
b) die Fumarsäure in dem geschmolzenen Xylitol dispergiert, wobei man eine Mischung erhält, und dann
c) die Mischung auf eine Temperatur unter etwa 94 °C abkühlt und dann
d) kristallines Xylitol zu der Mischung gibt, bevor die Mischung fest geworden ist, und dann
e) die Mischung fest werden lässt.

5. Verfahren nach Anspruch 4, bei dem man das kristalline Xylitol in Schritt c) zugibt, nachdem die Mischung auf etwa 88-92 °C abgekühlt ist und/oder während die Mischung noch flüssig ist.

6. Verfahren nach einem der vorhergehenden Ansprüche, bei dem die Schmelzverarbeitungstemperatur mindestens 91 °C und weniger als 180 °C, vorzugsweise mindestens 91 °C, aber nicht mehr als 120 °C, beträgt.

7. Feste Zusammensetzung, die durch das Verfahren nach einem der Ansprüche 1 bis 6 erhältlich ist.

8. Feste Dispersion einer organischen Säure in Xylitol, wobei die organische Säure aus Citronensäure, Fumarsäure, Weinsäure, Äpfelsäure, Bernsteinsäure und Ascorbinsäure ausgewählt ist.

9. Feste Dispersion nach Anspruch 8, wobei es sich bei der organischen Säure um Fumarsäure handelt.

10. Feste Dispersion nach den Ansprüchen 8 bis 9, die in Form von mehreren Einheiten vorliegt.

11. Feste pharmazeutische Zusammensetzung, umfassend:
a) mindestens einen therapeutischen Wirkstoff, der aus einem Calcium- oder Magnesiumsalz oder einer Kombination davon ausgewählt ist, und
b) die feste Zusammensetzung nach Anspruch 7 oder die feste Dispersion nach den Ansprüchen 8 bis 10.

12. Feste pharmazeutische Zusammensetzung nach Anspruch 11, wobei das Calciumsalz aus Calcium-acetat, Calciumcarbonat, Calciumchlorid oder einer Kombination davon ausgewählt ist.

13. Feste pharmazeutische Zusammensetzung nach Anspruch 12, wobei es sich bei dem Calciumsalz um Calciumacetat handelt und/oder es sich bei dem Magnesiumsalz um Magnesiumcarbonat handelt.

14. Feste pharmazeutische Zusammensetzung nach den Ansprüchen 10 bis 13, die in Form eines Granulats, eines Direktgranulats, eines Pulvers für die orale Anwendung, einer Tablette, einer Kautablette oder einer Schmelztablette vorliegt.

15. Feste pharmazeutische Zusammensetzung nach den Ansprüchen 10 bis 14 zur Verwendung bei der Behandlung oder Prävention von mit chronischer Niereninsuffizienz assoziierter Hyperphosphatämie bei Hämodialyse- oder Peritonealdialyse-Patienten (CKD Stadium 5) oder bei Patienten mit CKD Stadium 4 oder früher.

## Revendications

1. Procédé de préparation d'une composition solide comprenant un acide organique, le procédé comprenant une étape visant à disperser l'acide organique dans du xylitol fondu de manière à former un mélange, puis à laisser le mélange se solidifier, l'acide organique étant fourni sous forme de poudre.

2. Procédé selon la revendication 1, l'acide organique étant choisi parmi l'acide citrique, l'acide fumarique, l'acide tartrique, l'acide malique, l'acide succinique et l'acide ascorbique.

3. Procédé selon la revendication 2, l'acide organique étant l'acide fumarique.

4. Procédé selon la revendication 3, comprenant les étapes suivantes
a) faire fondre le xylitol,
b) disperser l'acide fumarique dans le xylitol fondu de manière à former un mélange, puis
c) refroidir le mélange à une température inférieure à environ 94 °C, puis
d) ajouter du xylitol cristallin au mélange avant que le mélange ne se soit solidifié, puis
e) laisser le mélange se solidifier.

5. Procédé selon la revendication 4, le xylitol cristallin étant ajouté à l'étape c) après que le mélange est refroidi à environ 88-92 °C et/ou pendant que le mélange est encore liquide.

6. Procédé selon l'une quelconque des revendications précédentes, la température de traitement à l'état fondu étant d'au moins 91 °C et inférieure à 180 °C, de préférence d'au moins 91 °C mais pas supérieure à 120 °C.

7. Composition solide pouvant être obtenue par le procédé selon l'une quelconque des revendications 1 à 6.

8. Dispersion solide d'un acide organique dans du xylitol, l'acide organique étant choisi parmi l'acide citrique, l'acide fumarique, l'acide tartrique, l'acide malique, l'acide succinique et l'acide ascorbique.

9. Dispersion solide selon la revendication 8, l'acide organique étant l'acide fumarique.

10. Dispersion solide selon les revendications 8 à 9, se présentant sous la forme d'unités multiples.

11. Composition pharmaceutique solide comprenant :
a) au moins un ingrédient thérapeutiquement actif choisi parmi un calcium ou un sel de magnésium ou une combinaison de ceux-ci, et
b) la composition solide selon la revendication 7 ou la dispersion solide selon les revendications 8 à 10.

12. Composition pharmaceutique solide selon la revendication 11, le sel de calcium étant choisi parmi l'acétate de calcium, le carbonate de calcium, le chlorure de calcium ou une combinaison de ceux-ci.

13. Composition pharmaceutique solide selon la revendication 12, le sel de calcium étant de l'acétate de calcium et/ou le sel de magnésium étant le carbonate de magnésium.

14. Composition pharmaceutique solide selon les revendications 10 à 13, se présentant sous la forme de granulés, de granulés à mettre directement dans la bouche, d'une poudre par voie orale, d'un comprimé, d'un comprimé à mâcher ou d'un comprimé à désintégration par voie orale.

15. Composition pharmaceutique solide selon les revendications 10 à 14, destinée à être utilisée dans le traitement ou la prévention de l'hyperphosphatémie associée à une insuffisance rénale chronique chez des patients hémodialysés ou en dialyse péritonéale (stade 5 de la maladie rénale chronique) ou chez des patients atteints d'insuffisance rénale chronique au stade 4 ou avant.
